# EUROPEAN PATENT APPLICATION

(11) **EP 1 437 410 A1**
(43) Date of publication of application: **14.07.2004**
(21) Application number: 02767954.7
(22) Date of filing: 12.09.2002
(51) Int. Cl.: C12N 15/53, C12N 9/02

(54) **NOVEL SELENOCYSTEIN-CONTAINING PROTEINS**

(30) Priority: 13.09.2001 JP 2001278749
(71) Applicant: Juridical Foundation, The Chemo-Sero-Therapeutic Research Institute, Kumamoto-shi, Kumamoto 860-8568 (JP)
(72) Inventor: KAMINAKA, Sara, Kikuchi-gun, Kumamoto 861-1102 (JP); KAMINAKA, Kazuyoshi, Kikuchi-gun, Kumamoto86 1-1102 (JP); HIRASHIMA, Masaki, Kikuchi-gun, Kumamoto 861-1102 (JP); MAEDA, Hiroaki, Kumamoto-shi, Kumamoto 860-0076 (JP); NOZAKI, Chikateru, Kumamoto-shi, Kumamoto 862-8001 (JP); TAKAHASHI, Kazuhiko, Sapporo-shi, Hokkaido 001-0037 (JP)
(74) Representative: VOSSIUS & PARTNER
(86) International application number: PCT/JP2002/009313
(87) International publication number: WO 2003/029469

(57) **Abstract**

A novel selenocysteine-containing protein is provided which is a substantial entity having an enzymatic activity, typically an activity to reduce peroxide phospholipids. There are provided a gene consisting of a coding sequence for a protein not containing selenocysteine in which the codon for selenocysteine is introduced at a desired position and a selenocysteine insertion sequence resided at the 3'-end of said coding sequence and a protein expressed therefrom, typically a selenocysteine-containing protein having an activity to reduce peroxide phospholipids which is prepared by introducing selenocysteine into albumin and as well as a gene encoding said protein. A novel anti-oxidative substance is provided which can be applied for prevention of worsening, prophylaxis or treatment of various diseases associated with oxidative stress.

SEQUENCE LISTING
<110> JURIDICAL FOUNDATION THE CHEMO-SERO-THERAPEUTIC RESEARCH INSTITUTE

<120> Novel protein containing serenocystein

<130> 663387

<150> JP 2001-278749
<151> 2001-09-13

<160> 11

<210> 1
<211> 52
<212> DNA
<213> Artificial Sequence

<220>
<223> Primer, PS1

<400> 1
ccgctcgaga agcttggcac gaggcaggcc cgttggaagt ggttgtgaca ac 52

<210> 2
<211> 105
<212> DNA
<213> Artificial Sequence

<220>
<223> Primer, PS2

<400> 2
ggaagatctg gatccgcggc cgctgagcat gctgaacaat aaagacacac acttgaaagg 60
ttttaaaatt gcatttttat tgaatttatt tggacaaatc cgtac 105

<210> 3
<211> 35
<212> DNA
<213> Artificial Sequence

<220>
<223> Primer, AlbF

<400> 3
cctcgagaaa agagatgcac acaagagtga ggttg 35

<210> 4
<211> 30
<212> DNA
<213> Artificial Sequence

<220>
<223> Primer, AlbR

<400> 4

ccgaattcgt tataagccta aggcagcttg                                    30

<210> 5
<211> 117
<212> DNA
<213> Artificial Sequence

<220>
<223> Primer, PH1

<400> 5
ccgctcgaga agcttggcac aatgaagtgg gtaaccttta tttcccttct tttctcttt      60
agctcggctt attccagggg tgtgtttcgt cgagatgcac acaagagtga ggttgct       117

<210> 6
<211> 115
<212> DNA
<213> Artificial Sequence

<220>
<223> Primer, PH3

<400> 6
cggaattcgg atcctctaga ctaaattggg gagtatgtcc tattttaaat atttaattca      60
gatcctcttc tgagatgagt ttttgttcta agcctaaggc agcttgactt gcagc         115

<210> 7

<211> 101

<212> DNA

<213> Artificial Sequence

<220>

<223> Primer, PH4(C34S)

<400> 7

cacaattttc agctgactca tcagcaacac atgtttttgc aaattcagtt acttcattca 60

ctaattttac atgatcttca aatggtgact gctgaagata c 101

<210> 8

<211> 101

<212> DNA

<213> Artificial Sequence

<220>

<223> Primer, PH5(C34U)

<400> 8

cacaattttc agctgactca tcagcaacac atgtttttgc aaattcagtt acttcattca 60

ctaattttac atgatcttca aatggtcact gctgaagata c 101

<210> 9

<211> 585

<212> PRT

<213> Human

<220>
<223> Albumin with Cys to serenocystein mutation at 34 position
<223> Xaa represents selenocysteine

<400> 9
Asp Ala His Lys Ser Glu Val Ala His Arg Phe Lys Asp Leu Gly
1               5                   10                  15
Glu Glu Asn Phe Lys Ala Leu Val Leu Ile Ala Phe Ala Gln Tyr
                20                  25                  30
Leu Gln Gln Xaa Pro Phe Glu Asp His Val Lys Leu Val Asn Glu
                35                  40                  45
Val Thr Glu Phe Ala Lys Thr Cys Val Ala Asp Glu Ser Ala Glu
                50                  55                  60
Asn Cys Asp Lys Ser Leu His Thr Leu Phe Gly Asp Lys Leu Cys
                65                  70                  75
Thr Val Ala Thr Leu Arg Glu Thr Tyr Gly Glu Met Ala Asp Cys
                80                  85                  90
Cys Ala Lys Gln Glu Pro Glu Arg Asn Glu Cys Phe Leu Gln His
                95                  100                 105
Lys Asp Asp Asn Pro Asn Leu Pro Arg Leu Val Arg Pro Glu Val
                110                 115                 120
Asp Val Met Cys Thr Ala Phe His Asp Asn Glu Glu Thr Phe Leu
                125                 130                 135
Lys Lys Tyr Leu Tyr Glu Ile Ala Arg Arg His Pro Tyr Phe Tyr
                140                 145                 150
Ala Pro Glu Leu Leu Phe Phe Ala Lys Arg Tyr Lys Ala Ala Phe
                155                 160                 165

Thr Glu Cys Cys Gln Ala Ala Asp Lys Ala Ala Cys Leu Leu Pro
                170                 175                 180
Lys Leu Asp Glu Leu Arg Asp Glu Gly Lys Ala Ser Ser Ala Lys
                185                 190                 195
Gln Arg Leu Lys Cys Ala Ser Leu Gln Lys Phe Gly Glu Arg Ala
                200                 205                 210
Phe Lys Ala Trp Ala Val Ala Arg Leu Ser Gln Arg Phe Pro Lys
                215                 220                 225
Ala Glu Phe Ala Glu Val Ser Lys Leu Val Thr Asp Leu Thr Lys
                230                 235                 240
Val His Thr Glu Cys Cys His Gly Asp Leu Leu Glu Cys Ala Asp
                245                 250                 255
Asp Arg Ala Asp Leu Ala Lys Tyr Ile Cys Glu Asn Gln Asp Ser
                260                 265                 270
Ile Ser Ser Lys Leu Lys Glu Cys Cys Glu Lys Pro Leu Leu Glu
                275                 280                 285
Lys Ser His Cys Ile Ala Glu Val Glu Asn Asp Glu Met Pro Ala
                290                 295                 300
Asp Leu Pro Ser Leu Ala Ala Asp Phe Val Glu Ser Lys Asp Val
                305                 310                 315
Cys Lys Asn Tyr Ala Glu Ala Lys Asp Val Phe Leu Gly Met Phe
                320                 325                 330
Leu Tyr Glu Tyr Ala Arg Arg His Pro Asp Tyr Ser Val Val Leu
                335                 340                 345
Leu Leu Arg Leu Ala Lys Thr Tyr Glu Thr Thr Leu Glu Lys Cys
                350                 355                 360
Cys Ala Ala Ala Asp Pro His Glu Cys Tyr Ala Lys Val Phe Asp
                365                 370                 375
Glu Phe Lys Pro Leu Val Glu Glu Pro Gln Asn Leu Ile Lys Gln

                380                 385                 390
Asn Cys Glu Leu Phe Glu Gln Leu Gly Glu Tyr Lys Phe Gln Asn
                395                 400                 405
Ala Leu Leu Val Arg Tyr Thr Lys Lys Val Pro Gln Val Ser Thr
                410                 415                 420
Pro Thr Leu Val Glu Val Ser Arg Asn Leu Gly Lys Val Gly Ser
                425                 430                 435
Lys Cys Cys Lys His Pro Glu Ala Lys Arg Met Pro Cys Ala Glu
                440                 445                 450
Asp Tyr Leu Ser Val Val Leu Asn Gln Leu Cys Val Leu His Glu
                455                 460                 465
Lys Thr Pro Val Ser Asp Arg Val Thr Lys Cys Cys Thr Glu Ser
                470                 475                 480
Leu Val Asn Arg Arg Pro Cys Phe Ser Ala Leu Glu Val Asp Glu
                485                 490                 495
Thr Tyr Val Pro Lys Glu Phe Asn Ala Glu Thr Phe Thr Phe His
                500                 505                 510
Ala Asp Ile Cys Thr Leu Ser Glu Lys Glu Arg Gln Ile Lys Lys
                515                 520                 525
Gln Thr Ala Leu Val Glu Leu Val Lys His Lys Pro Lys Ala Thr
                530                 535                 540
Lys Glu Gln Leu Lys Ala Val Met Asp Asp Phe Ala Ala Phe Val
                545                 550                 555
Glu Lys Cys Cys Lys Ala Asp Asp Lys Glu Thr Cys Phe Ala Glu
                560                 565                 570
Glu Gly Lys Lys Leu Val Ala Ala Ser Gln Ala Ala Leu Gly Leu
                575                 580                 585

<210> 10

<211> 620

<212> PRT

<213> Human

<220>

<223> HindIII-BamHI insertion sequence in C34U expression vector

<223> Xaa represents selenocysteine

<400> 10

Met Lys Trp Val Thr Phe Ile Ser Leu Leu Phe Leu Phe Ser Ser

1               5                   10                  15

Ala Tyr Ser Arg Gly Val Phe Arg Arg Asp Ala His Lys Ser Glu

                20                  25                  30

Val Ala His Arg Phe Lys Asp Leu Gly Glu Glu Asn Phe Lys Ala

                35                  40                  45

Leu Val Leu Ile Ala Phe Ala Gln Tyr Leu Gln Gln Xaa Pro Phe

                50                  55                  60

Glu Asp His Val Lys Leu Val Asn Glu Val Thr Glu Phe Ala Lys

                65                  70                  75

Thr Cys Val Ala Asp Glu Ser Ala Glu Asn Cys Asp Lys Ser Leu

                80                  85                  90

His Thr Leu Phe Gly Asp Lys Leu Cys Thr Val Ala Thr Leu Arg

                95                  100                 105

Glu Thr Tyr Gly Glu Met Ala Asp Cys Cys Ala Lys Gln Glu Pro

                110                 115                 120

Glu Arg Asn Glu Cys Phe Leu Gln His Lys Asp Asp Asn Pro Asn

                125                 130                 135

Leu Pro Arg Leu Val Arg Pro Glu Val Asp Val Met Cys Thr Ala

                140                 145                 150
Phe His Asp Asn Glu Glu Thr Phe Leu Lys Lys Tyr Leu Tyr Glu
                155                 160                 165
Ile Ala Arg Arg His Pro Tyr Phe Tyr Ala Pro Glu Leu Leu Phe
                170                 175                 180
Phe Ala Lys Arg Tyr Lys Ala Ala Phe Thr Glu Cys Cys Gln Ala
                185                 190                 195
Ala Asp Lys Ala Ala Cys Leu Leu Pro Lys Leu Asp Glu Leu Arg
                200                 205                 210
Asp Glu Gly Lys Ala Ser Ser Ala Lys Gln Arg Leu Lys Cys Ala
                215                 220                 225
Ser Leu Gln Lys Phe Gly Glu Arg Ala Phe Lys Ala Trp Ala Val
                230                 235                 240
Ala Arg Leu Ser Gln Arg Phe Pro Lys Ala Glu Phe Ala Glu Val
                245                 250                 255
Ser Lys Leu Val Thr Asp Leu Thr Lys Val His Thr Glu Cys Cys
                260                 265                 270
His Gly Asp Leu Leu Glu Cys Ala Asp Asp Arg Ala Asp Leu Ala
                275                 280                 285
Lys Tyr Ile Cys Glu Asn Gln Asp Ser Ile Ser Ser Lys Leu Lys
                290                 295                 300
Glu Cys Cys Glu Lys Pro Leu Leu Glu Lys Ser His Cys Ile Ala
                305                 310                 315
Glu Val Glu Asn Asp Glu Met Pro Ala Asp Leu Pro Ser Leu Ala
                320                 325                 330
Ala Asp Phe Val Glu Ser Lys Asp Val Cys Lys Asn Tyr Ala Glu
                335                 340                 345
Ala Lys Asp Val Phe Leu Gly Met Phe Leu Tyr Glu Tyr Ala Arg
                350                 355                 360

Arg His Pro Asp Tyr Ser Val Val Leu Leu Leu Arg Leu Ala Lys
                365                 370                 375
Thr Tyr Glu Thr Thr Leu Glu Lys Cys Cys Ala Ala Ala Asp Pro
                380                 385                 390
His Glu Cys Tyr Ala Lys Val Phe Asp Glu Phe Lys Pro Leu Val
                395                 400                 405
Glu Glu Pro Gln Asn Leu Ile Lys Gln Asn Cys Glu Leu Phe Glu
                410                 415                 420
Gln Leu Gly Glu Tyr Lys Phe Gln Asn Ala Leu Leu Val Arg Tyr
                425                 430                 435
Thr Lys Lys Val Pro Gln Val Ser Thr Pro Thr Leu Val Glu Val
                440                 445                 450
Ser Arg Asn Leu Gly Lys Val Gly Ser Lys Cys Cys Lys His Pro
                455                 460                 465
Glu Ala Lys Arg Met Pro Cys Ala Glu Asp Tyr Leu Ser Val Val
                470                 475                 480
Leu Asn Gln Leu Cys Val Leu His Glu Lys Thr Pro Val Ser Asp
                485                 490                 495
Arg Val Thr Lys Cys Cys Thr Glu Ser Leu Val Asn Arg Arg Pro
                500                 505                 510
Cys Phe Ser Ala Leu Glu Val Asp Glu Thr Tyr Val Pro Lys Glu
                515                 520                 525
Phe Asn Ala Glu Thr Phe Thr Phe His Ala Asp Ile Cys Thr Leu
                530                 535                 540
Ser Glu Lys Glu Arg Gln Ile Lys Lys Gln Thr Ala Leu Val Glu
                545                 550                 555
Leu Val Lys His Lys Pro Lys Ala Thr Lys Glu Gln Leu Lys Ala
                560                 565                 570
Val Met Asp Asp Phe Ala Ala Phe Val Glu Lys Cys Cys Lys Ala

                575                 580                 585
Asp Asp Lys Glu Thr Cys Phe Ala Glu Glu Gly Lys Lys Leu Val
                590                 595                 600
Ala Ala Ser Gln Ala Ala Leu Gly Leu Glu Gln Lys Leu Ile Ser
                605                 610                 615
Glu Glu Asp Leu Asn
                620

<210> 11
<211> 2769
<212> DNA
<213> Human

<220>
<223> HindIII-BamHI insertion sequence in C34U expression vector

<400> 11
   aagcttggca caatgaagtg ggtaaccttt atttcccttc tttttctctt tagctcggct 60
   tattccaggg gtgtgtttcg tcgagatgca cacaagagtg aggttgctca tcggtttaaa 120
   gatttgggag aagaaaattt caaagccttg gtgttgattg cctttgctca gtatcttcag 180
   cagtgaccat ttgaagatca tgtaaaatta gtgaatgaag taactgaatt tgcaaaaaca 240
   tgtgttgctg atgagtcagc tgaaaattgt gacaaatcac ttcataccct ttttggagac 300
   aaattatgca cagttgcaac tcttcgtgaa acctatggtg aaatggctga ctgctgtgca 360
   aaacaagaac ctgagagaaa tgaatgcttc ttgcaacaca aagatgacaa cccaaacctc 420
   ccccgattgg tgagaccaga ggttgatgtg atgtgcactg cttttcatga caatgaagag 480
   acatttttga aaaaatactt atatgaaatt gccagaagac atccttactt ttatgccccg 540
   gaactccttt tctttgctaa aaggtataaa gctgctttta cagaatgttg ccaagctgct 600
   gataaagctg cctgcctgtt gccaaagctc gatgaacttc gggatgaagg gaaggcttcg 660
   tctgccaaac agagactcaa gtgtgccagt ctccaaaaat ttggagaaag agctttcaaa 720
   gcatgggcag tagctcgcct gagccagaga tttcccaaag ctgagtttgc agaagtttcc 780
   aagttagtga cagatcttac caaagtccac acggaatgct gccatggaga tctgcttgaa 840
   tgtgctgatg acagggcgga ccttgccaag tatatctgtg aaaatcaaga ttcgatctcc 900
   agtaaactga aggaatgctg tgaaaaacct ctgttggaaa aatcccactg cattgccgaa 960
   gtggaaaatg atgagatgcc tgctgacttg ccttcattag ctgctgattt tgttgaaagt 1020
   aaggatgttt gcaaaaacta tgctgaggca aaggatgtct tcctgggcat gtttttgtat 1080
   gaatatgcaa gaaggcatcc tgattactct gtcgtgctgc tgctgagact tgccaagaca 1140
   tatgaaacca ctctagagaa gtgctgtgcc gctgcagatc ctcatgaatg ctatgccaaa 1200
   gtgttcgatg aatttaaacc tcttgtggaa gagcctcaga atttaatcaa acaaaattgt 1260
   gagctttttg agcagcttgg agagtacaaa ttccagaatg cgctattagt tcgttacacc 1320
   aagaaagtac cccaagtgtc aactccaact cttgtagagg tctcaagaaa cctaggaaaa 1380
   gtgggcagca aatgttgtaa acatcctgaa gcaaaaagaa tgccctgtgc agaagactat 1440
   ctatccgtgg tcctgaacca gttatgtgtg ttgcatgaga aaacgccagt aagtgacaga 1500
   gtcaccaaat gctgcacaga atccttggtg aacaggcgac catgcttttc agctctggaa 1560
   gtcgatgaaa catacgttcc caaagagttt aatgctgaaa cattcacctt ccatgcagat 1620
   atatgcacac tttctgagaa ggagagacaa atcaagaaac aaactgcact tgttgagctc 1680
   gtgaaacaca agcccaaggc aacaaaagag caactgaaag ctgttatgga tgatttcgca 1740
   gcttttgtag agaagtgctg caaggctgac gataaggaga cctgctttgc cgaggagggt 1800
   aaaaaacttg ttgctgcaag tcaagctgcc ttaggcttag aacaaaaact catctcagaa 1860
   gaggatctga attaaatatt taaaatagga catactcccc aatttagtct agacacaatt 1920
   tcatttccag catttttata aactaccaaa ttagtgaacc aaaaatagaa attagatttg 1980
   tgcaaacatg gagaaatcta ctgaattggc ttccagattt taaattttat gtcatagaaa 2040
   tattgactca aaccatattt tttatgatgg agcaactgaa aggtgattgc agcttttggt 2100
   taatatgtct ttttttttct ttttccagtg ttctatttgc tttaatgaga atagaaacgt 2160
   aaactatgac ctaggggttt tctgttggat aattagcagt ttagaatgga ggaagaacaa 2220
   caaagacatg ctttccattt tttcctttac ttatctctca aaacaatatt actttgtctt 2280
   ttcaatcttc tacttttaac taataaaata agtggatttt gtattttaag atccagaaat 2340
   acttaacacg tgaatatttt gctaaaaaag catatataac tattttaaat atccatttat 2400
   cttttgtata tctaagactc atcctgattt ttactatcac acatgaataa aggcctttgt 2460
   atctttcttt ctctaatgtt gtatcatact cttctaaaac ttgagtggct gtcttaaaag 2520
   atataagggg aaagataata ttgtctgtct ctatattgct tagtaagtat ttccatagtc 2580
   aatgatggtt taataggtaa accaaaccct ataaacctga cctcctttat ggttaatact 2640
   attaagcaag aatgcagtac agaattggat acagtacgga tttgtccaaa taaattcaat 2700
   aaaaatgcaa ttttaaaacc tttcaagtgt gtgtctttat tgttcagcat gctcagcggc 2760
   cgcggatcc 2769

## Description

### TECHNICAL FIELD

The present invention relates to a novel protein belonging to the field of a medical drug, particularly, to a novel substance having an enzymatic activity prepared by introducing selenocysteine into a protein not containing selenocysteine. More particularly, the present invention relates to a gene consisting of a coding sequence for a protein not containing selenocysteine in which the codon for selenocysteine is introduced at an appropriate position and a selenocysteine insertion sequence (SECIS) resided at the 3' end of said coding sequence, and a protein expressed therefrom. Preferably, the present invention relates to a selenocysteine-containing protein having an activity to reduce peroxide phospholipids which is prepared by introducing selenocysteine into albumin as well as a gene encoding the same. The present invention allows for provision of a medicament for prevention of worsening, prophylaxis or treatment of various diseases associated with oxidative stress.

### Background Art

Recently, there has successively been revealed correlation between oxidative lesions of biomolecules by active oxygen species, free radical, and various diseases, e.g. aging, inflammation, autoimmune disease, carcinogenesis, ischemic reperfusion injury, neurodegeneration, arterial sclerosis, diabetes, cataract or muscular atrophy. In the state of these diseases, a variety of anti-oxidative agents are acting within the living body so as to counteract oxidative stress, cause of diseases. Known anti-oxidative agents include a protein such as superoxide dismutase (SOD), catalase, glutathione peroxidase (GPx) or ceruloplasmin, and a lower molecule such as glutathione (GSH), vitamins E and C, and the like.

The anti-oxidative activity is also known in albumin. Human serum albumin (HSA) is initially biosynthesized as a preproprotein of 609 residues, from which the N-terminal eighteen residues are removed by signal peptidase and then the subsequent six residues are further cleaved off while passing through a secretion pathway, resulting in mature albumin of 585 amino acid residues present in plasma. Since albumin accounts for about 50 to 60% of a whole serum protein (7.5 to 8.0 g/100 ml) and about 40% of a total amount thereof within the living body is present in plasma while the remaining 60% is within the extracellular matrix, the anti-oxidative activity of albumin is regarded as quantitatively being significant within the living body.

It is believed that the anti-oxidative ability of albumin is exerted mainly through buffering action to oxidation-reduction. The conditions within the living body and cells are aerobic with a rather low oxygen level. Life phenomenon occurs under such reductive circumstances and hence requires subtle balance in oxidoreductive conditions. For example, GSH possesses a single SH group within the molecule and utilizes this to let the intracellular circumstances under reductive conditions. Likewise, albumin has a free (reductive) cysteine at the 34th position (Cys34), counted from the N-terminal of mature albumin, and like GSH, utilizing its extensive amount, prevents the interior circumstances of the living body from being inclined to be acidic so as to keep the inside of the living body under reductive conditions.

Recently, it has been revealed that albumin has an enzymatic activity to reduce peroxide phospholipids and its active center is Cys34 (R. HURST et al., Biochem. J., 338, 723 (1999)). In normal adult male, about 75% of albumin has free Cys34. This ratio, however, is known to change with aging in such a manner that free Cys34 decreases with aging. Also, in some diseases e.g. chronic hepatic disease or renal failure, it is observed that reductive albumin is extremely reduced. It is also indicated that reductive albumin is decreased in patients suffering from sepsis, a kind of oxidative stress diseases, as compared to healthy adult, with decreased activity to reduce peroxide phospholipids. Importance of albumin in maintaining homeostasis or in oxidative stress diseases is thus recognized.

### DISCLOSURE OF THE INVENTION

Viewing that there is only one reductive SH group per one molecule of albumin, however, administration of an extensive amount of albumin will be necessary for enhancing clinical effects. Administration of a large amount of albumin preparations is restricted both from ethical and physiological point of views and adverse side effects resulting from extensive administration are also known. For example, when albumin is administered at 4 g/dl or more, an ability of the living body to albumin biosynthesis may be inhibited and immune suppression may occasionally be induced. Besides, infusion at a high concentration or in a large amount may become a burden to the heart causing lowering of blood pressure or even heart failure. In order to avoid these adverse side effects, there exists need for albumin with enhanced anti-oxidative activity and for functional modification of albumin. In practice, however, no such albumin with improved function is known up till the present.

In describing the specification, we referred to the following literatures: "All about anti-oxidative substance", ed. by Toshikazu Yoshikawa, SENTAN IGAKU-SHA; "Clinical albuminology", ed. by Akiharu Watanabe, MEDICAL VIEW CO., LTD.; "Multi-functional protein: Serum albumin", Seiich Era, KYORITSU SHUPPAN CO., LTD.; "All about Albumin", T. Peters, JR, ACADEMIC PRESS; "Great Medical Dictionary" CD-ROM, NANZANDO Co., Ltd.; and "Up-to-date Great Medical Dictionary", CD-ROM, 2nd Ed., Ishiyaku Publishers, Inc.

Under the circumstances, the present inventors have investigated to develop a novel medicament for treating anti-oxidative stress, especially one for treating diseases where peroxide lipids are involved, and as a result, have succeeded in introducing into human albumin selenocysteine (hereinafter referred to as "Sec"), an amino acid resulting from replacement of sulfur (S) in Cys with selenium (Se), in place of Cys34 and found that the resultant modified albumin had a high activity to reduce peroxide phospholipids that has hitherto not been reported. The present inventors have thus found that a novel substance with a high anti-oxidative activity could be produced by introducing Sec into a protein not containing Sec and based on this finding completed the present invention.

### BRIEF DESCRIPTION OF DRAWINGS

Fig. 1 shows a map of a plasmid in which human selenoprotein P cDNA is inserted wherein poly A addition signal is indicated by 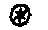 .
Fig. 2 illustrates steps of construction of a modified albumin gene.
Fig. 3 illustrates steps of construction of a modified albumin gene.
Fig. 4 is a photograph showing Western blot of expressed modified albumin in which each lane in (A) and (B) are as follows: (A) Lane 1: C34C, Lane 2: C34U, Lane 3: intact CHO, Lane 4: HSA (The Chemo-Sero-Therapeutic Research Institute), and Lane 5: BSA; (B) Lane 6: C34C, Lane 7: C34U, Lane 8: intact CHO, Lane 9: HSA (The Chemo-Sero-Therapeutic Research Institute), and Lane 10: BSA.
Fig. 5 shows an elution pattern obtained when culture supernatant of modified albumin (C34U)-expressing cells is passed through an anti-HSA antibody column.
Fig. 6 is a photograph showing an electrophoretic pattern in SDS-PAGE of each of the modified albumins prepared in accordance with the present invention in which eluted fractions are shown by 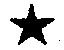 .
Fig. 7 shows change in an activity to reduce peroxide phospholipids of the modified albumins with lapse of time wherein the upper and lower graphs are presented for PCOOH and PCOH levels, respectively.

### BEST MODE FOR CARRYING OUT THE INVENTION

A novel protein with an enzymatic activity according to the present invention is a recombinant Sec-containing protein which is prepared on the basis of a protein not containing Sec through substitution of one or more Cys in said protein with Sec or insertion or substitution of one or more Sec and which has an enzymatic activity. Preferably, said enzymatic activity is an activity to reduce peroxide phospholipids and the protein not containing Sec, on the basis of which the recombinant protein is prepared, is albumin. Moreover, the present invention provides a gene encoding said recombinant Sec-containing protein as well as a process for preparing said recombinant Sec-containing protein by using said gene.

For enhancement of function of Cys34, the present inventors have devised to alter "S" in Cys34 into "Se". Se belongs to the group of oxygen (O) and S in the periodic table positioned under these elements and has a quite similar property to that of S except for their atomic radius with substantially no difference. A Sec selenol resulting from replacement of S in Cys with Se is capable of reducing peroxides via nucleophilic reaction but with much higher reducing activity than that of S-possessing Cys. Besides, a Sec selenol has a pKa value of 5.24 whereas a Cys thiol has a pKa value of 8.25. Namely, at around neutrality such as in the circumstances within the living body, a Cys thiol hardly dissociates whereas a Sec selenol is mostly ionized. Focusing on such distinctive property between Se and S, the present inventors considered it possible to enhance the anti-oxidative function of albumin.

For altering S in Cys into Se, there are mainly three approaches. First approach utilizes a protein synthesis system such as those in the living body or cells, or cell-free system. As described above, both Se and S have a quite similar physical property to each other. Hence, in the presence of an excess amount of inorganic Se, an amino acid biosynthesis system may randomly incorporate Se in place of S. Thus, when an excess amount of Se source such as inorganic Se is either directly administered to the living body or added to a cell culture system of eukaryotic cells such as animal cells or yeast cells, incorporation of Se in place of S for Cys occurs at a certain probability to thereby produce Sec. This approach however is disadvantageous in that incorporation is hard to control as other S-possessing amino acids such as methionine may also incorporate Se to produce e.g. selenomethionine.

In second approach, Sec is introduced by the chemical reaction. Targeting serine, this approach utilizes chemical modification to alter this amino acid into Sec. Specifically, a protein of interest is treated with PMSF (phenylmethanesulfonyl fluoride) and then with H₂Se, or with NaHSe and H₂O₂, to convert serine into Sec (Z. P. Wu and D. Hilvert, 111, 4513 (1989)). With this approach, serine in a light chain of GSH-binding antibody has been changed to Sec (G. M. Luo et al., Biochem. Biophys. Res. Commun. 198, 1240 (1994)), or the 221st serine as the active center of a serine protease subtilisin has been altered to Sec (Z. P. Wu and D. Hilvert, 112, 5647 (1990)), both producing an enzyme having an activity to reduce peroxides. However, this approach is also disadvantageous in that only serine can be targeted and a site-specific modification is difficult to make.

Third approach utilizes a gene recombination technique. Sec is encoded by the termination codon UGA and translated in a special mechanism (J. F. Atkins and R. F. Gesteland, Nature, 407, 463 (2000)). In the 3'-untraslated region (3' UTR) of selenoprotein mRNA, there is a sequence called "Selenocysteine Insertion Sequence (SECIS)" that can form a stem-loop structure. Since translation terminates upon removal of SECIS, it is known that this sequence is essential for recognition of the codon UGA as insertion of selenocysteine but not as termination (M. J. Berry et al., Nature, vol. 353, p. 273 (1991); M. J. Berry et al., EMBO J., vol. 12, p. 3315 (1993)).

It is thought that at least three factors are involved in the UGA translation mechanism in eukaryotes, i.e. Sec-tRNA with an anticodon to UGA (B. J. Lee et al., J. Biol. Chem., 264, 9724 (1989)), an elongation factor (eEFSec) that specifically binds to this tRNA (D. Fagegaltier et al., EMBO J., 19, 4796 (2000)), and an SECIS-binding protein (SBP2)(P. R. Copeland et al., EMBO J., 19, 306 (2000)) . When translation starts from AUG and a polypeptide is synthesized up to UGA, Sec-tRNA is provided from eEFSec that is bound with SECIS-SBP2 complex. Sec is then bound to the polypeptide chain, translation continued even after insertion of Sec and terminates at the termination codon other than UGA. Third approach utilizes the biosynthesis mechanism of Sec-containing protein. All these three approaches may be used for introducing Sec but the last one may be most suitable as allowing for Sec introduction assuredly and site-specifically as compared to the former two approaches.

The present inventors thus employed the third approach to devise a method for preparing a protein having an anti-oxidative activity that has Sec at a desired position by constructing a gene consisting of a coding sequence of a protein not containing Sec in which TGA codon for Sec is introduced at a desired position and SECIS sequence at the 3'-end of said coding sequence and by expressing said gene in animal cells. More specifically, the method of the present invention may be applied to any protein not containing Sec as far as it has an activity to reduce peroxides via thiol donor such as GSH, including even an artificially produced protein such as abzyme, an antibody having an enzymatic activity.

The most suitable protein for the purpose includes albumin. The position at which Sec is introduced in such a protein may be anywhere provided that the codon is placed in frame with the amino acid sequence with no limitation to the number of Sec to be introduced. Sec is most preferably introduced at such an amino acid as the active center, e.g. Cys or serine, with the 34th Cys being the most suitable in case of albumin. An exemplary amino acid sequence of Sec-containing mature albumin with such a desirable sequence is indicated in SEQ ID NO: 9. An SECIS sequence may be introduced at any position as far as it is at the 3'-end of the amino acid coding sequence. Any kind of SECIS sequences may be used that are derived from a gene of selenoproteins in any number or in any combination thereof with the 3'-untranslated region of selenoprotein P being preferable.

Any expression control sequence such as promoter or enhancer or any element necessary for gene expression such as poly A addition sequence may be used in the method if it is one for animal cells. The thus designed and constructed gene may be introduced into any animal cells by the conventional methods such as lipofectin or electroporation. After introduction, however, animal cells must be cultured with supplement of Sec sources such as selenite or other selenoproteins.

The method as described above allows for production of a novel Sec-containing protein as a novel functional protein. The Sec-containing protein produced by said method is a novel protein having an anti-oxidative activity, especially when derived from albumin, a novel protein having an activity to reduce peroxide phospholipids in the presence of a thiol donor.

### INDUSTRIAL APPLICABILITY

A novel protein in accordance with the present invention may be used as a medicament for the treatment and prophylaxis of any disease where oxidative lesions of biomolecules due to active oxygen species, free radical, so-called oxidative stress, is involved. Especially, the protein may suitably be used as a medicament for the treatment of the state of diseases including aging, inflammation, carcinogenesis, autoimmune diseases, ischemic reperfusion injury, neurodegeneration, arterial sclerosis, and the like. Such a disease include, for instance, a disease where reperfusion injury is observed such as myocardial infarction, cerebral infarction or organ transplantation; a disease where cell death or oxidative stress is involved such as AIDS or neurodegenerative diseases, e.g. Parkinson disease, Alzheimer disease, spinocerebellar degeneration; immune diseases such as asthma or rheumatism; and an inflammatory disease such as sepsis. Besides, viewing that the protein contains Sec, it may also be applied to a disease related to tissues and organs that require a high level of selenium, e.g. cranial nerves, heart, muscle, immune system, and genital organs. Moreover, it may further be applied to a disease state where the conventional albumin preparations have been used, such as hemorrhagic of traumatic shock, highly invasive operation, cirrhosis, and nephrosis.

The present invention is explained in more detail by means of the following Examples in which reagents from Wako Pure Chemical Industries, Ltd., TAKARA SHUZO CO., Ltd., Toyobo, New England BioLabs, Pharmacia, BioRad, Sigma, and GIBCO BRL were used.

### Example 1

### (Construction of modified albumin gene)

### (1) Vector containing selenoprotein P (SeP) 3'-untransnlated region

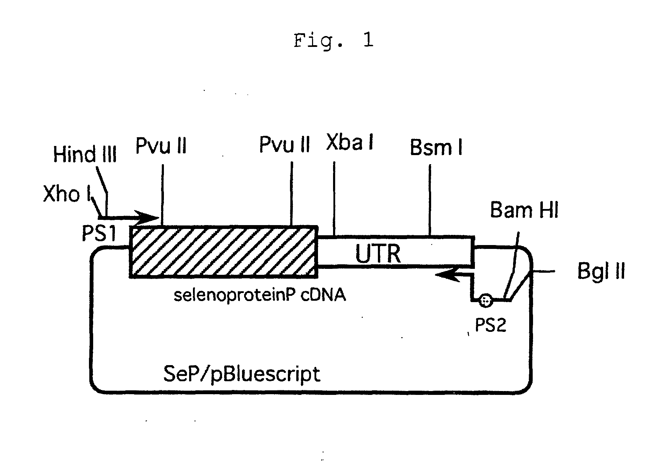

In order to introduce restriction enzyme XhoI/HindIII recognition sites at the 5'-end of human SeP cDNA and BamHI/BglII recognition sites and mouse selenoprotein P poly A addition signal at the 3'-end, PCR was performed using plasmid SeP/pBluescript with inserted human SeP cDNA (by courtesy of Dr. K. Takahashi, an assistant professor of pharmacy at Hokkaido University) as a template, with primers having the following sequences (Fig. 1):

PS1:5'CCGCTCGAGAAGCTTGGCACGAGGCAGGCCCGTTGGAAGTGGTTGTGACAAC

(SEQ ID NO: 1)

PS2:5'GGAAGATCTGGATCCGCGGCCGCTGAGCATGCTGAACAATAAAGACACACACT
TGAAAGGTTTTAAAATTGCATTTTTATTGAATTTATTTGGACAAATCCGTAC (SEQ
ID NO: 2).

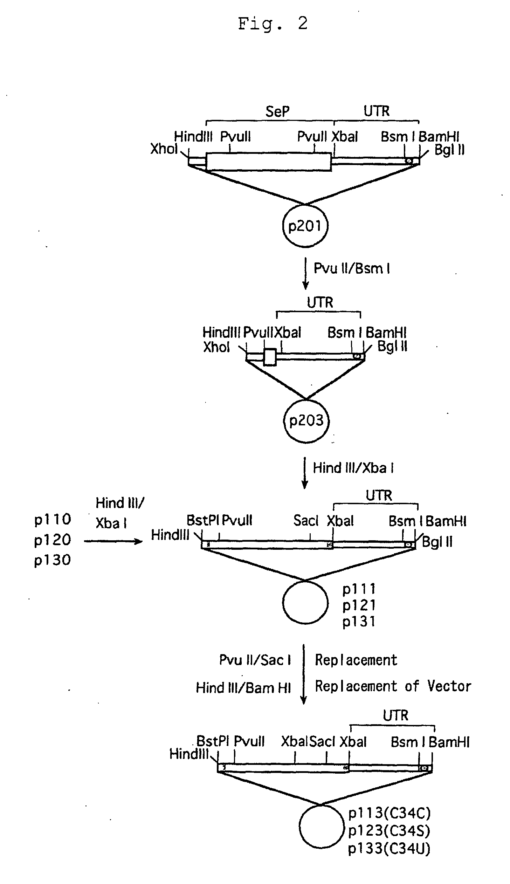

Using Astec program temp control system PC-800 for a thermal cycler and LATaq (TAKARA) for a DNA polymerase, PCR was performed 25 cycles of 96°C for 20 seconds and 68°C for 3 minutes and then 1 cycle of 68°C for 5 minutes, using a concentration of reagents in accordance with the protocol attached to the kit. The obtained amplified fragments of about 2 kbp were cloned into pCR2.1 vector in accordance with the protocol of TOPO TAcloning kit (Invitrogen). The obtained clones were analyzed for a DNA sequence of about 200 bp at the 5'-end of the inserted fragment up to the PvuII recognition site and a DNA sequence of about 100 bp at the 3'-end of the inserted fragment up to the BsmI recognition site, using M13 reverse and T7 as a primer. Based on the analysis, DNA clones with correctly inserted sequence were selected, digested with XhoI/BglII, and the resulting fragment of about 2.1 kbp was incorporated into pSP72 vector (p201; Fig. 2). Of two inner fragments obtained after digestion of SeP/pBluescript with PvuII/BsmI, the PvuII/BsmI fragment at the 3'-end (0.9 kbp) was inserted into a fragment from p201 after PvuII/BsmI digestion containing a vector portion (p203; Fig. 2).

### (2) Construction of human plasma albumin (HSA) expression vector C34C in which c-myc is inserted

For preparing HSA cDNA, PCR was performed under the same conditions as aforementioned with cDNA as a template that was prepared from Hepatocyte mRNA (Sawaday Technology) using a single-chain cDNA synthesis kit (Pharmacia), with primers having the following sequences:

AlbF: 5'CCTCGAGAAAAGAGATGCACACAAGAGTGAGGTTG (SEQ ID NO: 3)

AlbR: 5'CCGAATTCGTTATAAGCCTAAGGCAGCTTG (SEQ ID NO: 4).

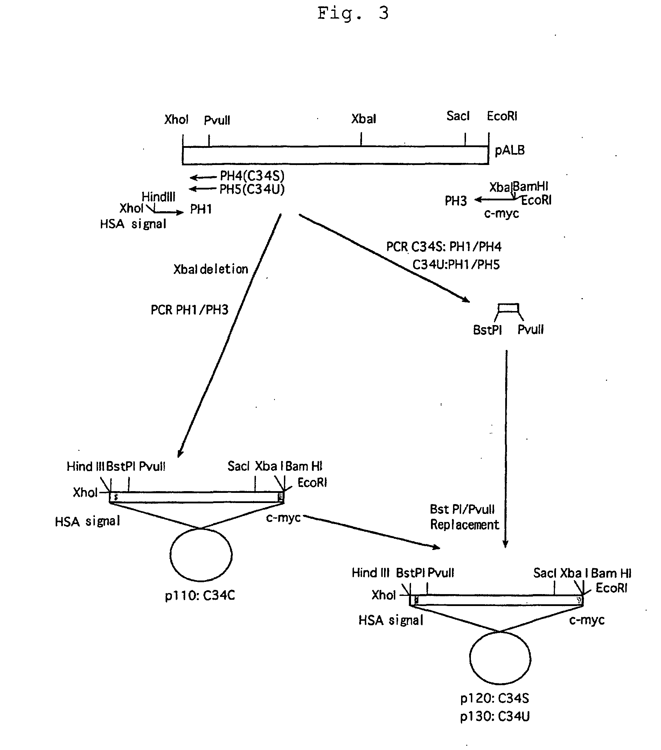

The obtained PCR fragments were digested with XhoI/EcoRI, the resulting digested fragments were cloned into XhoI/EcoRI site of pBluescriptII (pALB; Fig. 3) and a full-length thereof was sequenced to confirm that no error such as frame shift error was contained therein.

Initially, XbaI site within pALB was deleted by XbaI digestion and treatment with T4 polymerase leaving blunt-ends, followed by self-ligation. Subsequently, in order to add XhoI/HindIII recognition sites and HSA signal sequence at the 5'-end of HSA cDNA and a portion of c-myc gene as a flag, a portion of 3'-untranslated region of SeP gene and XbaI/BamHI/EcoRI recognition sites at the 3'-end, PCR was performed with pALB as a template and LATaq polymerase using the following primers (Fig. 3) under the same conditions as aforementioned except for 30 cycles of 96°C for 20 seconds and 68°C for 2 minutes.

PH1:5'CCGCTCGAGAAGCTTGGCACAATGAAGTGGGTAACCTTTATTTCCCTTCTTTT
TCTCTTTAGCTCGGCTTATTCCAGGGGTGTGTTTCGTCGAGATGCACACAAGAGTGAGG
TTGCT (SEQ ID NO: 5)

PH3:5'CGGAATTCGGATCCTCTAGACTAAATTGGGGAGTATGTCCTATTTTAAATATT
TAATTCAGATCCTCTTCTGAGATGAGTTTTTGTTCTAAGCCTAAGGCAGCTTGACTTGC
AGC (SEQ ID NO: 6)

The obtained amplified fragments of about 1.8 kbp were cloned into pCR2.1 vector. Based on the sequence analysis, a clone that had correct sequences both at the 5'-end ATG up to the PvuII site and at the 3'-end up to the SacI site was selected. This clone was digested with XhoI/EcoRI and the resulting fragment was inserted into pSP72. A PvuII/SacI digestion fragment from this plasmid was replaced with a PvuII/SacI fragment from pALB prior to PCR (p110).

p203 and p110 were digested with HindIII/XbaI and a fragment of about 1.9 kbp from p110 was inserted into a fragment of about 3.5 kbp from p203 (p111). A PvuII/SacI fragment within p111 was replaced with a PvuII/SacI fragment from pALB. The resulting plasmid was digested with HindIII/BamHI and the obtained HSA fragment of about 2.9 kbp with c-myc was inserted into HindIII/BamHI site of pCAG mcs (Japanese patent application No. 165249/1996 (republished WO97/46583) to produce C34C expression vector (p113).

### (3) Construction of HSA expression vectors C34S and C34U in which c-myc is inserted and the 34th Cys is altered to Ser or Sec

In order to add restriction enzyme XhoI/HindIII recognition sites and HSA signal sequence at the 5'-end of HSA cDNA and to alter the codon TGT for Cys34 about 110 bp downstream from ATG into the codon TCA for Ser or the codon TGA for Sec, PCR was performed with pALB as a template using the PH1 primer and the following primers (Fig. 3) under the same conditions as aforementioned.

PH4(C34S):5'CACAATTTTCAGCTGACTCATCAGCAACACATGTTTTTGCAAATTCA
GTTACTTCATTCACTAATTTTACATGATCTTCAAATGGTGACTGCTGAAGATAC (SEQ
ID NO: 7)

PH5(C34U):5'CACAATTTTCAGCTGACTCATCAGCAACACATGTTTTGCAAATTCA
GTTACTTCATTCACTAATTTTACATGATCTTCAAATGGTCACTGCTGAAGATAC (SEQ
ID NO: 8)

The obtained amplified fragments of about 200 bp were cloned into pCR2.1 vector. Based on the sequence analysis, clones that had correct sequences were selected. These clones were digested with restriction enzymes BstPI/PvuII and the resulting fragments were replaced for BstPI/PvuII digestion fragments of p110. The obtained clones were named as p120 (C34S) and p130 (C34U), respectively. p203, p120 and p130 were digested with HindIII/XbaI and each fragment of about 1.9 kbp from p120 and p130 was inserted into a fragment of about 3.5 kbp from p203 (p121, p131). Each PvuII/SacI fragment within p121 and p131 was replaced with a PvuII/SacI fragment from pALB. Each of the resulting plasmids was digested with HindIII/BamHI and each of the obtained fragments of about 2.9 kbp was inserted into HindIII/BamHI site of pCAG mcs to produce C34S and C34U expression vectors, p123 and p133, respectively. SEQ ID NO: 11 shows a DNA sequence of HindIII-BamHI insertion fragment of p133 whereas SEQ ID NO: 10 shows an amino acid sequence of an expression product thereof.

### Example 2

### (Expression of modified albumin gene)

### (1) Introduction of expression vector into cells

A DNA to be introduced was prepared as described below. E. coli JM109 (TOYOBO) cells were transformed with each of the constructed expression vectors C34C, C34S or C34U, shake-cultured on 250 ml LB medium (1% tryptone, 0.5% yeast extract, 1% NaCl) at 37°C overnight and plasmids were purified by alkali SDS. A portion (20 µg) of the obtained plasmid solution was taken and digested with PvuI to cleave the vector at a single point. After observing complete digestion of the vector by agarose gel electrophoresis, the plasmid vector was treated with phenol (phenol : CHCl₃ : isoamylalcohol = 25:24:1) and was subject to ethanol precipitation to remove proteins so as to extract nucleic acids, which were then dissolved in distilled water from which pyrogen was removed. The DNA fragment equivalent to 2 µg was used for transfection of cells per well (2 x 10⁵ cells).

Cells to which the DNA is to be introduced were prepared as described below. CHO cells (Chinese Hamster Ovary cells; Dainippon Pharmaceutical Co. Ltd.) were grown on RPMI1640 (Sigma) containing 600 ng/ml SeP fragment (Japanese Patent Application No. 347863/1998 (Republished publication WO00/31131)) and 10% FBS (Fetal Bovine Serum, HyClone) in a laboratory dish. The cells were peeled off by the action of trypsin, suspended and, after determining cell count, a cellular solution in said medium was prepared at 1.0 x 10⁵ cells/ml. The cells were inoculated on 6-well test plate for culture (Φ 35 mm) at 2 ml/well and cultured at 37°C in the presence of 5% CO₂ overnight.

For introduction of the gene into CHO cells, Trans-IT LT1 (Mirus) was used as a lipofectin solution for transfection. A mixture of 200 µl serum free medium Opti-MEM (GIBCO BRL) and 10 µl LT1 was gently blended in a 4 ml polystyrene tube and left to stand at room temperature for 5 minutes. A solution of the DNA fragment to be introduced equivalent to 2 µg was then added and the mixture was gently blended and left to stand at room temperature for 5 minutes (DNA/lipofectin complex). The cells were plated on a 6 well plate and culture supernatant was removed by suction and replaced with the same fresh medium. A total amount of the DNA/lipofectin complex solution was added to the cells in one well. The cells in said well were gently agitated and cultured at 37°C in the presence of 5% CO₂ for 6 to 8 hours. The culture supernatant was replaced with the same fresh medium.

### (2) Production of stable transformant cells

After cell culture under the conditions described above for 3 days, the culture medium was replaced with RPMI1640 containing 0.4 mg/ml G418 (GIBCO BRL), 10% FBS and 600 ng/ml SeP fragment. Culture was continued for 3 days while replacing the culture medium everyday and then for 8 days without changing the culture medium. After the cells were densely sheeted on one well of a 6 well plate, they were peeled off by trypsin treatment and stored in liquid nitrogen.

### (3) Preparation of expressed products

Each of the stable transformant cells were suspended in RPMI1640 containing 10% FBS and 600 ng/ml SeP fragment and plated in a 15 cm laboratory dish for culture. After culture, the dish was washed with PBS and the culture medium was replaced with ASF containing 600 ng/ml SeP fragment. With exception of the parent cells, 0.4 mg/ml G418 was added. After three-day culture, 160 ml culture supernatant was recovered and the cells were removed with 0.22 µm filter. With a ultrafiltration membrane of 10000 cut for molecular size, C34C, C34S and C34U were concentrated to 138.5-fold, 80-fold and 131-fold, respectively, and used as a sample in the assay described below.

For control of culture supernatant, the parent cells were inoculated on RPMI1640 containing 10% FBS and 600 ng/ml SeP fragment and culture supernatant was obtained in the same manner using ASF containing 600 ng/ml SeP fragment as a serum free medium and finally concentrated to 130-fold.

### (4) Immunoprecipitation - Western blot

Immunoprecipitation was performed with anti-HSA affinity Sepharose (BETHYL Laboratories, Inc.) as described below for Sample No. 1: a concentrate (138.5-fold) of culture supernatant from C34C-introduced cells; No. 2: a concentrate (131-fold) of culture supernatant from C34U-introduced cells; No. 3: a concentrate (130-fold) of culture supernatant from parent CHO cells; No. 4: HSA; and No. 5: BSA.

Each of the samples was mixed with 20 µl anti-HSA affinity Sepharose with Tween-20 at a final concentration of 0.01% in a 1.5 ml test tube and the mixture was reacted at room temperature for 1 hour. The mixture was then centrifuged at 6000 rpm for 5 minutes. Precipitated gel was washed with PBS and then with PBS containing 0.1% Tween. To the precipitate obtained after centrifugation was added 2 x 20 µl SDS sample buffer (not containing a reducing agent) and the mixture was blended and heated to 100°C for 5 minutes. After centrifugation, culture supernatant was dispensed into two tubes at equivalent portions and used as a sample in Western blot.

SDS-PAGE 12.5% acrylamide gel was prepared and each of the immunoprecipitated Samples No. 1 to No. 5 and molecular weight marker (Rainbow markers; Amersham) were electrophoresed on two gels with the same pattern. Gels after electrophoresis were used for Western blot. Using a blotting device (ATTO Bioscience, Inc.), transfer to PVDF membrane was performed in accordance with the attached protocol (each PVDF membrane is hereinafter referred to "A" and "B"). The PVDF membranes were immersed in 4% skimmed milk (DIFCO) and shook at 37°C for 30 minutes. As a primary antibody, either a biotin-labeled anti-c-myc antibody at a final concentration of 1 µg/ml for "A" or an HRP (Horse Radish Peroxidase)-labeled anti-HSA antibody at a final concentration of 0.2 µg/ml for "B", respectively, was dissolved in PBS containing 0.05% Tween and 4% skimmed milk, and each of the PVDF membranes was immersed in the resulting PBS and reacted at 37°C for 1 hour. After washing the membranes with PBS containing 0.05% Tween (PBST), the PVDF membrane "A" was immersed into 4% skimmed milk and shook at room temperature for 5 minutes. For an avidin-HRP label reagent, a solution of VECSTATIN (VECTOR; PK-6100) in PBST containing 0.4% skimmed milk was then prepared in accordance with the attached protocol. The PVDF membranes were immersed in the resulting solution and reacted at 37°C for 30 minutes. After washing with PBST, both "A" and "B" PVDF membranes were subject to chemical luminescence with ECL Plus (Amersham) in accordance with the attached protocol. An X-ray film (Konica) was exposed and developed by successively immersing into a developing solution and a fixing solution (both from Konica).

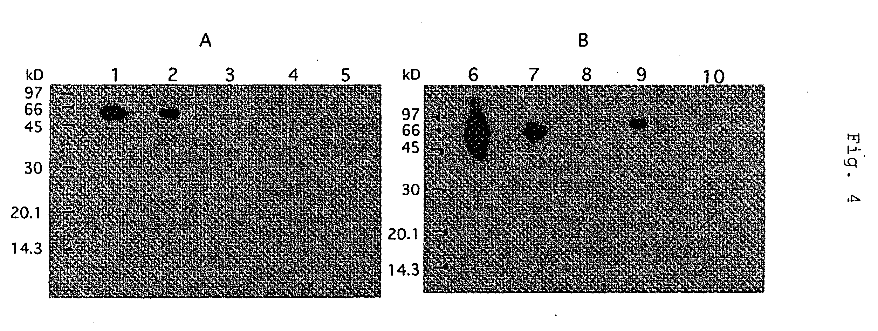

The results are shown in Fig. 4. In (A), lanes 1 and 2 indicate that each culture supernatant from the C34C-and C34U-introduced cells reacted with an ani-c-myc antibody, whereas no reaction could be observed for culture supernatant from the parent cells in lane 3, for HSA in lane 4, and for bovine serum albumin (BSA) in lane 5. On the other hand, in (B), lanes 6, 7 and 9 indicated that each culture supernatant from the C34C- and C34U-introduced cells reacted with an anti-HSA antibody whereas no reaction could be observed for culture supernatant from the parent cells in lane 8, and for bovine serum albumin in lane 10. Assessing from a size of the band detected in culture supernatant from the C34U-introduced cells, it was considered that translation did not terminate at the 34th Sec and hence Sec was inserted at the 34th position.

### (5) Detection by ELISA

An albumin content was determined in culture supernatant from each of the CHO cells where either C34C, C34U or C34S was introduced. For each of the concentrates, an albumin content as HSA was measured using an HSA quantitation kit (Human Albumin ELISA quantitation kit; BETHYL Laboratories, Inc.) in accordance with the attached protocol. As a result, it was found that C34C, C34U and C34S could all be detected as albumin as shown in Table 1.

**Table 1:**

| Expression level of modified albumin | | | | | |
|---|---|---|---|---|---|
| Sample | C34C | C34S | C34U | NC | C34U clone |
| Conc. fold | 138.5 | 80 | 131 | 1 | 1 |
| Conc. (ng/ml) | 61000 | 30300 | 1900 | 0 | ~37 |

### Example 3

### (Preparation of modified albumin)

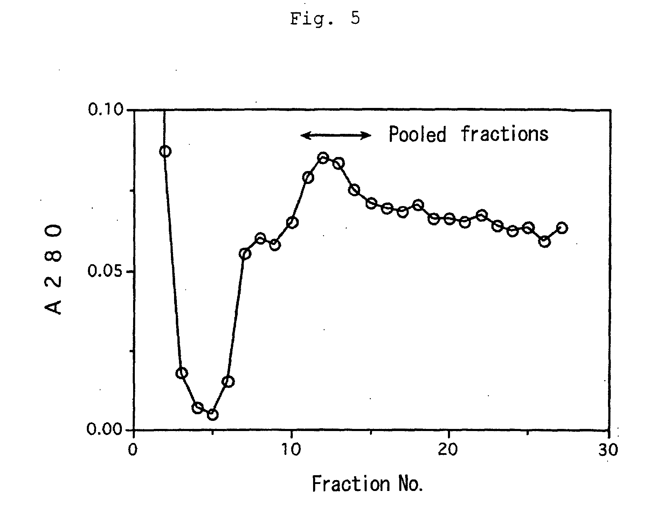

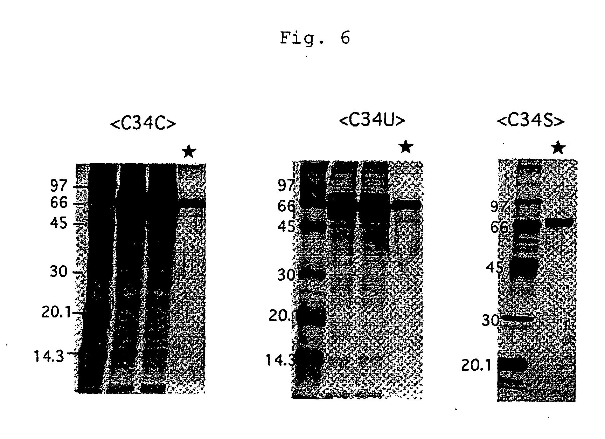

Each of the modified albumin-expressing CHO cells were cultured and expanded in RPMI1640 containing 10% FBS and plated on 31 laboratories dishes of 15 cm at 1.0 x 10⁵ cells/cm². On the next day, after washing the cellular surface with PBS, each 30 ml of ASF containing 1% FBS was added and the cells were cultured at 37°C (5% CO₂) for 5 days. Culture supernatant was recovered, scaled up to one liter with the medium and filtered through 0.45 µm filter. The obtained filtrate was used for application to a column. A column (diameter: 1.5 cm) was charged with 1 ml of an anti-HSA Sepharose and equilibrated with PBS. Equilibration was conducted with PBS while elution was performed with 0.1 M glycine, 0.1 M NaCl, pH 2.8. For elution, a test tube for collection has been charged with 120 µl of 1 M Tris, pH 8.0, per 1 ml of a fraction. All the eluates were filtered through 0.45 µm filter. One liter of the protein solution was applied to the anti-HSA column at a flow rate of 1.0 ml/min. and, after application was completed, the column was washed with 25 ml PBS. Subsequently, an elution buffer was passed through the column at the same flow rate and each 1 ml/fraction was collected. Absorbance at A280 was measured, 5 ml around the peak was pooled (Fig. 5), and concentrated up to 300 µl with Centricon 10 (MW 10,000 cut, Millipore). Fig. 6 shows electrophoretic patterns of SDS-PAGE for each of the concentrated samples in which a protein dying was made with a silver dye (KANTO KAGAKU).

### Example 4

### (Se content of modified albumin)

In order to investigate whether C34U modified albumin contains Se, an atomic absorption spectrometry was performed to determine Se content using Perkin-Elmer A Analyst 600 as an atomic absorption spectrometric device. A selenium standard solution (Nacalai Tesque, Inc.) 1000 ppm was used as a Se standard solution and diluted with a diluting solution (0.04% sodium deoxycholate, 0.01% Triton X-100 solution) to prepare a calibration curve. For C34U, a protein solution at a concentration of 15 µg/ml as albumin was diluted 2-fold with a dilution solution for atomic absorption spectrometry and used as a sample. As for C34C, however, a concentration of 15 µg/ml as albumin was less than a detection limit, and hence a protein solution at a concentration of 1.8 mg/ml was diluted in a similar manner to C34U and used as a sample. Likewise, for C34S, a protein solution at a concentration of 1.5 mg/ml was diluted in a similar manner to C34U and used as a sample. The results measured for each sample are shown in Table 2. As a result, C34U was shown to possess one Se per one albumin molecule as expected.

**Table 2:**

| Se content of each sample | | | | |
|---|---|---|---|---|
| Sample | Albumin conc. (µg/ml) | Volume (ml) | Selenium conc. (µg/L) | Selenium content (number/albumin molecule) |
| C34U | 14.7 | 0.3 | 15.38 | 0.9143 |
| C34C | 1850 | 0.3 | 0.8 | 0.0004 |
| C34S | 1500 | 0.2 | 0.32 | 0.0002 |

### Example 5

### (Activity of modified albumin to reduce peroxide phospholipids)

### (1) Substrate

Phosphatidylcholine hydroperoxide (PCOOH): L-α-phosphatidylcholine,β-linoleoyl-γ-palmitoil (PLPC, Sigma) 100 mg was dissolved in 500 ml of 0.2 M Tris-HCl, pH 8.8, containing 5 mM deoxycholate Na (Nacalai Tesque, Inc.) and thereto was added soybean lipoxydase (Biozyme laboratories) for reaction for 30 minutes. After the reaction was completed, the substrate was extracted with ethyl acetate, evaporated to dryness under reduced pressure, and then suspended in MeOH. The suspension was then applied to a column charged with TSKgel ODS-80Ts (Φ 8.0 x 250 mm, TOSOH) and HPLC purification was performed using MeOH/H₂O (93:7) as a mobile phase. A concentration of purified PCOOH was determined by iodometry using cumene hydroperoxide (Nacalai Tesque, Inc.) as a standard.

### (2) Preparation of reaction solution

A reaction solution of 0.1 M Tris-HCl, pH 7.4, containing 0.5 mM EDTA 3Na (Nacalai Tesque, Inc.), 10 mM NaN₃ (Nacalai Tesque, Inc.), 0.025% (v/v) Triton X-100 (Wako), 0.3 mM deoxycholate Na, and 2 mM glutathione (GSH; Nacalai Tesque, Inc.) was prepared and thereto was added each of the samples at each concentration as indicated below. After reaction at 37°C for 10 minutes, the substrate PCOOH was added at 60 nmol/ml to initiate the reaction.

| Sample conc.: | |
|---|---|
| C34U | 3.68 µg/ml |
| C34S | 188 µg/ml |
| C34C | 463 µg/ml |
| HSA (Japanese Red Cross Society) | 4000 µg/ml |

### (3) Preparation of sample for HPLC

After reaction at each point of 0, 2, 4, 8, and 24 hours, 30 µl was recovered from the reaction solution and suspended in 270 µl of 2-propanol. The suspension was then centrifuged at 10000 rpm at 4°C for 15 minutes and the obtained supernatant was used as a sample for HPLC.

### (4) PCOOH determination by HPLC

HPLC was performed with a column charged with TSKgel ODS-80Ts (Φ 4.6 x 250 mm, TOSOH) using CH₃CN/MeOH/H₂O (75:21:4) containing 10 mM coline chloride (Wako) as a mobile phase at a flow rate of 1.5 ml/min. The HPLC sample (50 µl) obtained in (3) was added to HPLC column. Detection of PCOOH and its reduced form PCOH was made by determining absorption at 234 nm, an absorption maximum of a conjugated diene. For a sample, a purified PCOOH and a reduction product thereof with sodium tetrahydroborate, PCOH, were used and PCOOH and PCOH in the reaction solution were determined on the basis of their peak area.

### (5) Results

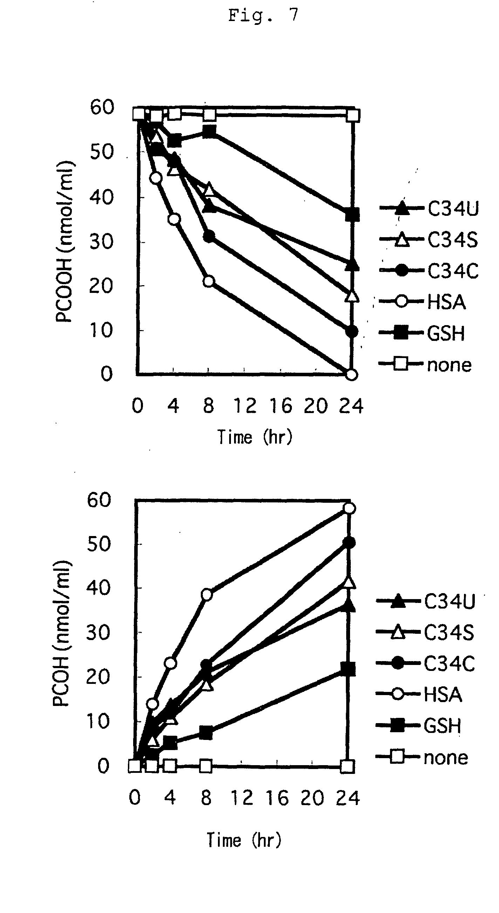

Fig. 7 shows change in levels of PCOOH and its reduced form, PCOH, with lapse of time. For every samples, decrease in PCOOH level and increase in PCOH level in a reaction time dependent manner was observed. Since at every time interval a decreased level of PCOOH and an increased level of PCOH are virtually consistent to each other, it is considered that PCOOH is reduced to PCOH.

An increased level of PCOH in case of addition of GSH control was subtracted from that in case of addition of each sample to draw an approximation curve, a slope of which was used to calculate an amount of PCOOH reduced per hour (nmol/hr/ml)(Table 3). This value gave a specific activity of each sample. The results are shown in Table 3. C34U had a specific activity of 8.4 nmol/min/mg which was about 120-fold and about 500-fold higher than those of control C34C (0.070 nmol/min/mg) and HSA (0.017 nmol/min/mg), respectively.

**Table 3:**

| Comparison of activity to reduce peroxide phospholipids in each sample | | | | | |
|---|---|---|---|---|---|
| Sample | Albumin conc. (µg/ml) | Amount of PCOOH reduced (nmol/hr/ml) | Specific activity (nmol/min/ mg) | Comparison of specific activity | |
| | | | | with C34C | with HSA |
| C34U | 3.68 | 1.86 | 8.42 | 120.4 | 495.3 |
| C34S | 188 | 1.40 | 0.12 | 1.7 | 7.1 |
| C34C | 463 | 1.92 | 0.070 | 1 | 4.1 |
| HSA | 4000 | 4.09 | 0.017 | 0.2 | 1 |

A novel protein obtained in accordance with the present invention is provided as a medicament for the treatment and prophylaxis of any disease where oxidative lesions of biomolecules due to active oxygen species, free radical, so-called oxidative stress, is involved, especially the state of diseases including aging, inflammation, carcinogenesis, autoimmune diseases, ischemic reperfusion injury, neurodegeneration, arterial sclerosis, and the like.

## Claims

1. A recombinant selenocysteine-containing protein which is prepared on the basis of a protein not containing selenocysteine through substitution of one or more cysteine in said protein with selenocysteine or insertion or substitution of one or more selenocysteine and which has an enzymatic activity.

2. The recombinant selenocysteine-containing protein of claim 1 wherein said enzymatic activity is an activity to reduce peroxide phospholipids.

3. The recombinant selenocysteine-containing protein of claim 1 or 2 wherein said protein not containing selenocysteine, on the basis of which the recombinant protein is prepared, is albumin.

4. The recombinant selenocysteine-containing protein of any one of claims 1 to 3 wherein said protein is a polypeptide chain having the amino acid sequence of SEQ ID NO: 9 or the amino acid sequence of SEQ ID NO: 9 in which one to several amino acids are deleted, substituted or added.

5. A gene coding for a recombinant selenocysteine-containing protein which is prepared on the basis of a protein not containing selenocysteine through substitution of one or more cysteine in said protein with selenocysteine or insertion or substitution of one or more selenocysteine and which has an enzymatic activity.

6. The gene of claim 5 which consists of a coding sequence for an amino acid sequence of a protein not containing selenocysteine in which the codon for selenocysteine is introduced at a desired position and a selenocysteine insertion sequence (SECIS sequence) resided at the 3'-untranslated region.

7. The gene of claim 6 wherein said SECIS sequence is derived from a selenoprotein P gene.

8. The gene of any one of claims 5 to 7 wherein a gene of said protein not containing selenocysteine is an albumin gene.

9. The recombinant selenocysteine-containing protein of claim 1 which is prepared by using the gene of any one of claims 5 to 8.

10. A process for preparing a recombinant selenocysteine-containing protein of claim 1 which comprises using the gene of any one of claims 5 to 8.
